Europäisches Patentamt

European Patent Office

Office européen des brevets

(11) Publication number: **0 271 109 A2**

# EUROPEAN PATENT APPLICATION

(21) Application number: 87118352.1

(22) Date of filing: 10.12.87

(51) Int. Cl.⁴: **G01N 21/35** , G01N 21/27 , A61B 5/00

(30) Priority: 11.12.86 US 939992

(43) Date of publication of application:
15.06.88 Bulletin 88/24

(84) Designated Contracting States:
DE FR GB

(71) Applicant: THE PERKIN-ELMER CORPORATION
761 Main Avenue
Norwalk Connecticut 06859-0181(US)

(72) Inventor: Mount, Bruce E.
21810 Manada Ct.
Diamond Bar, CA 91765(US)
Inventor: Becker, Douglas P.
731 Stillwater Lane
Anaheim, CA 91789(US)

(74) Representative: Patentanwälte Grünecker,
Kinkeldey, Stockmair & Partner
Maximilianstrasse 58
D-8000 München 22(DE)

(54) Respiratory CO2 detector circuit with high quality waveform.

(57) A respiratory $CO_2$ detector (10) comprising an infrared lamp source (44) and an infrared detector (50) responsive thereto forming an optical path for detecting the change in $CO_2$ concentration, or an obstruction in a cuvette (42). The output of the infrared detector (50) provides a continuous high and low voltage signal to be applied to a feedback control loop (12) and to an output circuit (14).

The feedback control loop (12) includes a peak detector (22), a contamination detector (24), a pulse-width modulator (26) and a low pass filter (28), the latter providing a DC bias on the infrared lamp (44). The peak detector (22) is connected to the pulse-width modulator (26) to maintain the lamp voltage constant and is connected to comparators (56,62) to compare both outputs of the peak and contamination detectors (22,24). The contamination detector (24) will respond to blockage in the cuvette (42).

The output control circuit (14) includes a sample-and-hold circuit (30) to direct the high and low voltage signal from the detector to a subtractor (32), the output of which is then inverted to provide a high quality waveform.

Fig. 2

RESPIRATORY CO$_2$ DETECTOR CIRCUIT
WITH HIGH QUALITY WAVEFORM

BACKGROUND OF THE INVENTION

This invention relates to CO$_2$ detectors for use as part of a respiratory breath monitoring system for patients, usually in a hospital environment, and is specifically directed to a respiratory CO$_2$ detector circuit which produces a high quality waveform from a cuvette for diagnostic purposes.

Still more specifically, this invention is an improvement over a respiratory CO$_2$ detector disclosed by D. Raemer in a U.S. patent application S/N 730,158 entitled "Respiration Detector" filed May 3, 1985.

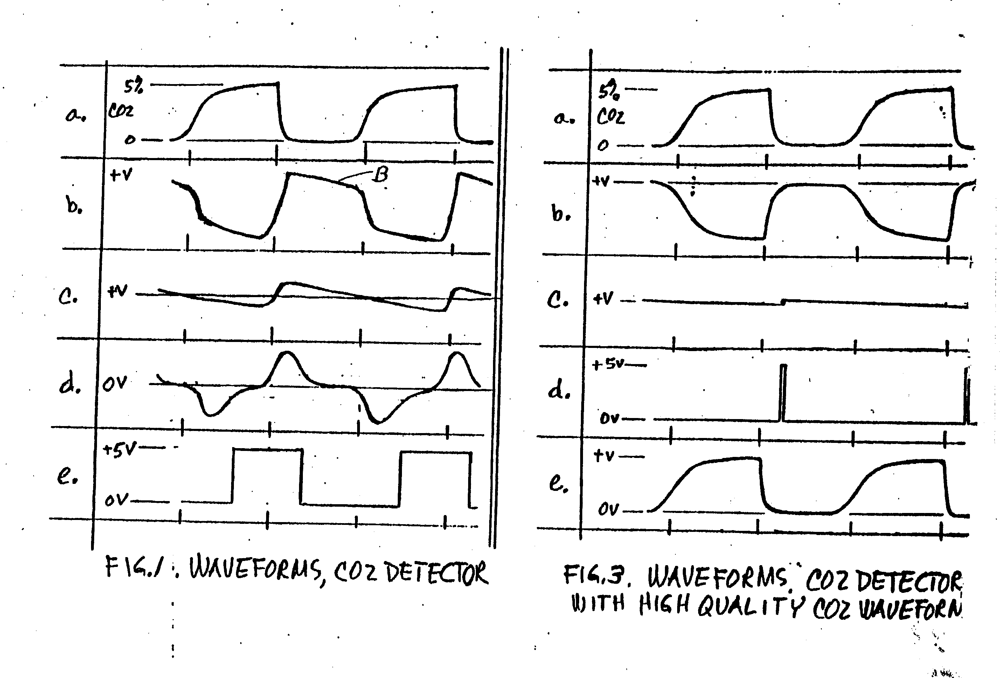

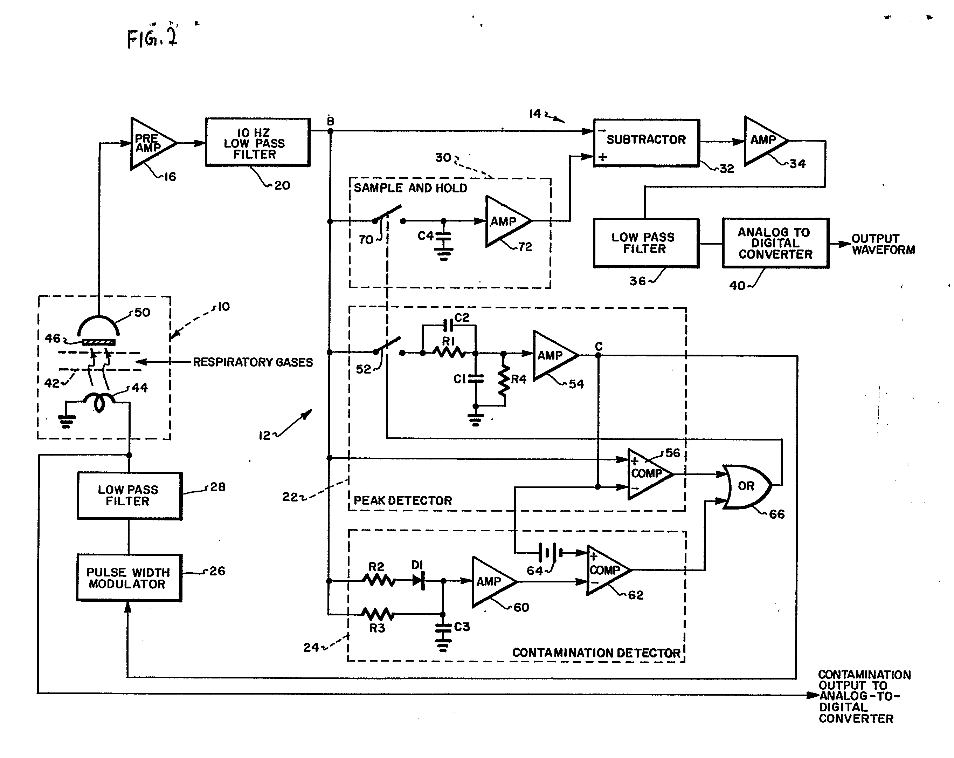

The Raemer patent application disclosed a respiratory CO$_2$ detector which included a sensor with a cuvette, a lamp as an infrared source, an infrared detector and amplifier for detecting the quantity of CO$_2$ gas in the cuvette, and suitable electronic amplification circuitry. In this system, an increase concentration of CO$_2$ gas in the cuvette resulted in an increased amount of energy absorption and less output from the infrared detector. Figure 1a illustrates a waveform of CO$_2$ gas in the cuvette. The high peaks represent approximately 5% concentration on exhalation and the valleys represent zero percent concentration on inhalation. Power for the infrared source was adjusted in the circuitry automatically depending upon the amount of attenuation in the optical path.

In this prior art scheme, no chopper or other reference was employed in order to keep manufacturing costs low. No attempt was made to preserve the quality of the $CO_2$ output waveform because only an alarm signal level was desired to indicate low $CO_2$ for the detection of apnea. Since no reference was employed, an artificial reference was developed by sensing the minimum $CO_2$ concentration from each breath and controlling the lamp voltage to maintain a constant voltage output from the detector amplifier. Thus, more lamp voltage was applied when the optical path was attenuated due to water vapor and/or particulate contamination from the gas sample which tended to maintain the output voltage sensitivity to $CO_2$ concentration in the dynamic breath waveform constant. The quality of the breath waveform was degraded because the system was designed to follow slow changes in $CO_2$ concentration and slow changes in optical path contamination. This caused a "droop" in the $CO_2$ waveform which was especially noticeable at lower breath rates of approximately four to six breaths per minute. This is shown in Figure 1b at "B". In order to refer the waveform to a baseline suitable for reliable operation of a threshold detector, the waveform was connected to a high pass filter, which caused further distortion. See Figures 1d and 1e where the waveform of 1d is the output from the high pass filter and 1e is the output of the threshold detector. This rendered the output waveform useless for diagnostic purposes. Nonetheless, the Raemer instrument did accomplish the purposes intended which were to (1) indicate by a flashing light when each expiratory breath occurred, and (2) sound an alarm when expiratory breaths were not present for a predetermined time.

When monitoring the concentration of $CO_2$ in a

patient's expired respiratory breath, it is important to have a high quality output waveform for diagnostic purposes.

This invention maintains the advantage of low manufacturing cost made possible by the Raemer invention, but enables the high quality $CO_2$ waveform to be obtained for diagnostic purposes.

Therefore, an object of this invention is to provide a respiratory $CO_2$ detector with

1) a high quality waveform without additional requirements for references for zero $CO_2$ or full scale $CO_2$,

2) a high quality waveform in which the minimum $CO_2$ concentration is referred to a stable zero baseline,

3) the ability to follow slow changes in $CO_2$ concentration with minimal waveform distortion.

4) the ability to compensate for slow changes in optical path transmission with minimal waveform distortion.

SUMMARY OF THE INVENTION

The respiratory $CO_2$ detector which meets the foregoing object comprises an infrared lamp source and an infrared detector responsive thereto forming an optical path for detecting the presence or absence of $CO_2$, or, an obstruction in a cuvette. The output of the infrared detector provides a voltage signal which is applied to a feedback control loop and to an output circuit.

The feedback control loop includes a preamplifier, a lowpass filter, a peak detector, a contamination detector and a pulse-width modulator, the latter providing a DC bias on the infrared lamp. The output of the peak detector is connected to the pulse-width modulator to maintain the lamp voltage constant and to comparators,one of which is necessary for operation of the peak detector, and the other is part of the contamination detector. The contamination detector will respond to blockage in the cuvette by readjusting the feedback loop to a normal condition.

The output control circuit includes a sample-and-hold circuit activated by the peak detector to direct the high and low voltage signal to a subtractor where the bias from the preamplifier output is eliminated. The output of the subtractor is then inverted to provide a high quality output waveform representative of the concentration of the $CO_2$ gas in the cuvette.

BRIEF DESCRIPTION OF THE DRAWINGS

Figure 1 is a series of waveforms of the prior art $CO_2$ detector,

Figure 2 is a block diagram of the respiratory $CO_2$ detector whose output is a high quality waveform useful for diagnostics, and

Figure 3 is a series of waveforms provided by the circuitry of Figure 2.

DETAILED DESCRIPTION
Circuit Description

In Figure 2 of the drawings, it can be seen that the $CO_2$ detector circuit of this invention includes a sensor 10 connected to a feedback control loop 12 and to an output circuit 14.

The feedback control loop 12 includes a preamplifier 16, a low pass filter 20, a peak detector 22, a contamination detector 24 and a pulse-width modulator 26 and a second low pass filter 28.

The output circuit 14, which is connected to the output of the low pass filter 20 includes a sample-and-hold circuit 30, a subtractor 32, an amplifier 34, a low pass filter 36 and an analog-to-digital converter 40. The output of the latter is the high quality output waveform.

The sensor 10 comprises a cuvette 42, a lamp 44 defining an optical path by radiating infrared energy tran versely through the cuvette, through an optical bandpass filter 46 and onto an infrared $CO_2$ detector 50.

The output of the lowpass filter 36 is the high quality waveform in analog form, while the analog digital converter is the digitized form of the waveform for acceptance by a microprocessor.

The output of the infrared detector 50 is connected to the preamplifier 16 whose output is connected to the low pass filter 20. The output of the low pass filter 20 is connected at point B to the peak detector 22, to the negative input of the subtractor 32 to the sample-and-hold circuit 30 and to the contamination detector 24.

The peak detector 22 is connected at point B in two places. First, an analog switch 52 is connected to point B. This switch 52 connects an RC network comprising resistor R1, capacitors C1 and C2 and resistor R4. Capacitor C2 is connected in parallel with resistor R1 and capacitor C1 is connected in series with resistor R2 and to ground. Resistor R4 is also connected to ground and in parallel with capacitor C1. The output of the RC network is connected to a buffer amplifier 54 and the output of the latter is connected to one input (-) of a comparator 56 and to the pulse-width modulator 26. The output of the pulse-width modulator is connected to the second low pass filter 28.

The second connection of the peak detector 22 to point B is directly to the other input (+) of the comparator 56.

The contamination detector 24 is also connected to point B and comprises a second RC network comprising resistor R2, R3, diode D1, and capacitor C3. Resistor R3 is connected in series with the capacitor C3 and the latter is connected to ground. A second resistor R2 and diode D1 are connected in parallel with resistor R3 and the output of this network is connected to the input of an amplifier 60. The output of amplifier 60 is connected to one input (-) of a second comparator 62. The other input (+) of comparator 62 is connected to the other input (-) of comparator 56 through a negative bias 64. The output of both comparators are connected to an OR gate 66 whose output is connected back to the switch 52 and to switch 70 of the sample-and-hold circuit 30 to be responsive thereto.

The output circuit 14 is connected to the low pass filter 20 at point B in two places. First, the analog switch 70 of the sample-and-hold circuit 30 connects point B to one side of a capacitor C4 and to the input of amplifier 72. The other side of capacitor C4 is connected to ground. Switch 70 is ganged with switch 52 and both are responsive to the output of the OR gate 66. The output of amplifier 72 is connected to one input of the subtractor 32. The other input of the subtractor is connected directly to point B. Again, the output of the subtractor 32 is connected to an input of inverting amplifier 34. The output of amplifier 34 is connected to low pass filter 36 and analog-to-digital converter 40.

Circuit Operation

In order to explain the operation of this invention, Figures 2 and 3 should be taken together.

Conventionally, the cuvette 42 is connected to a patient's breathing circuit to measure the $CO_2$ concentration of the expired breaths. A gas sample is drawn through the cuvette via a capillary tube from the cuvette to a vacuum source, and another capillary tube is connected from the cuvette to an adapter inserted in series with a conventional airway tube which is in turn connected from a respiratory ventilation device to the patient's airway. Figure 3a, like Figure 1a, shows a typical respiratory $CO_2$ concentration waveform and the actual gas concentration percentages found in respiratory breathing. Zero percent represents inhalation and the peak of 5%

represents exhalation of $CO_2$ by a patient.

The pulse-width modulator 26 has an internal reference voltage of +5 VDC which is internally compared to the voltage at point C (the output of amplifier 54) to maintain a DC level at point B. The low pass filter 28 provides DC voltage to lamp 44. The bandwidth of the optical bandpass filter 46 is optimized to be within the energy absorption band of $CO_2$ gas. The output voltage of the infrared detector 50 is proportional to the amount of radiation received from the lamp 44. This voltage is seen in Figure 3b and, as shown, decreases when $CO_2$ is present, or the optical path is obstructed such as by water droplets or particulate matter, and increases when the optical path is free of $CO_2$.

Thus, $CO_2$ gas in the cuvette is detected by the detector 50. The above-mentioned feedback control loop 12 is actually a gain control for the voltage at lamp 44 to keep the DC bias at point B constant.

Again, referring to Figure 3b, when the percentage of $CO_2$ is zero, or when the optical path is not otherwise obstructed, the output voltage at point B is a positive voltage of approximately +4 VDC. This voltage is applied directly to the positive input of the comparator 56. The output of the comparator 56 (Figure 3d) is applied to the OR gate 66 which momentarily closes the switch 52. (This is the situation also when the circuit is originally placed in operation). The closing of this switch allows the DC voltage at point B to charge the capacitors C1 and C2 in the peak detector 22 to the minimum $CO_2$ concentration voltage (or maximum DC voltage). The switches open when the $CO_2$ concentration begins to increase above zero volts.

During the increase of the percentage of $CO_2$ to a peak as shown in Figure 3a, the voltage at point B gradually drops to a low voltage. This drop in voltage is applied to the positive input of the comparator 56 which voltage level is less than the voltage at the negative input to this comparator 56 by reason of the fact that the RC network of the peak detector maintained a high voltage (with a slight gradual drop) at the output of the amplifier 54. This is shown in Figure 3c. This same voltage, being applied to the input of the pulse-width modulator 26, provides only a gradual decrease in voltage at the lamp 44.

When the $CO_2$ decreases with a consequent increase in voltage as shown in Figures 3a and 3b, this voltage increase is again applied to the comparator 56, momentarily closing the switch 52 and again charging the RC network as before.

This cyclical operation of the feedback control loop maintains the voltage on the lamp 44 and the DC reference at point B constant so that the waveform at point B is representative of the condition of the cuvette.

During the same cyclical operation, capacitor C4 in the sample-and-hold circuit 30 is charged when the switch 70 is closed and is allowed to discharge when the switch 70 is open. This provides an output signal at the positive input of the subtractor 32. Since the negative input of the subtractor 32 is connected directly to point B, the subtractor serves to subtract the DC bias at point B to provide a zero VDC baseline for the waveform. Since this waveform is inverted, the output of the subtractor 32 is applied to the inverting amplifier 34, the output of which ultimately shows a high quality waveform referred to a zero baseline as shown in Figure 3e.

During the cyclical operation of the feedback control loop, the high and low voltages at point B are also applied to the RC network of the contamination detector 24. The capacitor C3 is charged during high voltage at point B and allowed to discharge during low voltage at point B and the output of the RC network is applied to amplifier 60 and then to the negative input of comparator 62. The positive input of comparator 62 is the output from amplifier 54 of the peak detector 22, less a negative DC bias 64. Therefore, when the DC output of the contamination detector amplifier 60 falls below the DC voltage at comparator 62 positive input, the output of the comparator 62 will switch from zero VDC to +12 VDC, causing switches 70 and 52 to close momentarily until capacitor C3 again charges to a higher DC voltage, bringing comparator 62 negative input more positive than the positive input. Since the discharge time constant of the RC network of the peak detector is many times longer than the discharge time constant of the RC network of the contamination detector 24, the function of the contamination detector 24 is muted until such time as the contamination in the cuvette causes the voltage at point B to remain lower than the output of the peak detector amplifier 54, minus a DC bias, for a period of time beyond the time constant of the contamination detector whereupon the contamination detector will close the switches 52 and 70 driving the voltage at the output of the peak detector amplifier 54 to a low level. At this time, the feedback loop automatically tries to increase the lamp voltage to maintain the voltage at point B at +4 volts. If the lamp voltage increases beyond a predetermined level due to excessive contamination, external processing circuitry can be employed to sound an alarm.

The peak detector RC network charge time constant is approximately 13 sec and the discharge time constant is approximately 20,000 sec. The contamination detector RC network charge time constant is a few milliseconds and the discharge time constant is approximately 100 sec. The system response time when blockage occurs in the cuvette is at least equal to or less than 30 sec.

It should be apparent that the high quality waveform of this invention can be analyzed by computer processing to provide alarms when the expired breath $CO_2$ concentration falls below a predetermined level, for

0 271 109

example, 1% to 2%.

CLAIMS:

1. A $CO_2$ detector including a cuvette, an infrared lamp responsive to the voltage applied thereto, and an infrared detector and detector preamplifier having a reference (baseline) voltage, said lamp being positioned to illuminate an optical path through said cuvette so that infrared detector will produce a signal representative of the quantity of $CO_2$ in said cuvette, the improvement comprising,

first means responsive to said signal to maintain the baseline voltage (minimum $CO_2$ concentration) of the detector preamplifier output at a nearly constant value,

second means responsive to said signal to return the detector preamplifier baseline voltage to a reference level if the optical path is partially blocked, and

means for providing a high quality output signal waveform corresponding to the $CO_2$ concentration changes occuring within said cuvette.

2. The detector as claimed in Claim 1 further including a signal which can be used for warning if contamination is present.

3. The detector as claimed in Claim 1 wherein said first means includes a peak detector whose output is connected through a pulse-width modulator and low pass filter to said lamp.

4. The detector as claimed in Claim 3 wherein said second means includes a contamination detector whose output is connected to the input of said peak detector to change the last voltage level sensed by said peak detector to a reference (baseline) voltage level when the optical path has become partially occluded.

5. The detector as claimed in Claim 4 wherein said peak detector has an RC network with a relatively short charging time and a long discharge time constant, and wherein said contamination detector has an RC network with a relatively short charging time constant, and a shorter discharge time than the peak detector whereby the output signal from said peak detector may be interrupted and returned to said reference (baseline) voltage level.

6. The detector as claimed in Claim 5 wherein a pulse-width modulator is connected between the output of said peak detector to provide a predetermined voltage level baseline at the output of said preamplifier.

7. The detector as claimed in Claim 6 wherein the output of said preamplifier is connected to a sample-and-hold circuit and to a subtractor whereby said subtractor eliminates the baseline voltage provided by said pulse-width modulator and returns the output waveform from said preamplifier to a zero baseline for a high quality output waveform.

·8. The combination claimed in Claim 7 wherein a warning signal may be taken at the output of said pulse-width modulator and low pass filter (lamp voltage).

9. A $CO_2$ detector for monitoring a patient's breath inhalation and exhalation including a cuvette connected to the patient's airway, an infrared lamp responsive to the voltage applied thereto, and an infrared detector and preamplifier, said lamp being positioned to provide an optical path through said cuvette so that the infrared detector will produce an electrical waveform representative of the quantity of $CO_2$ in said cuvette, the improvement comprising,

a peak detector with an RC network connected to said preamplifier and whose output is connected to said lamp through a pulse-width modulator and lowpass filter,

a contamination detector with an RC network connected to said preamplifier and whose output is connected to the input of said peak detector,

the output of said peak detector and said contamination detector being continuously monitored by comparators with the discharge time of the RC network of said contamination detector having a time constant shorter than the discharge time constant of the RC network of said peak detector to override the RC network of the peak detector and raise the voltage on said lamp in the event the preamplifier output remains below the contamination detector positive input voltage.

10. The $CO_2$ detector as claimed in Claim 9, wherein said pulse-width modulator causes the voltage level of said preamplifier to remain at a baseline DC level and wherein said voltage level is reduced by a subtractor in order that the waveform output from said subtractor will represent a waveform representative of the concentration of $CO_2$ in said cuvette, but inverted.

11. The $CO_2$ detector as claimed in Claim 10 wherein said last mentioned waveform is inverted for a high quality waveform output.

12. The $CO_2$ detector as claimed in Claim 11 wherein the output of said detectors create a signal for triggering an alarm representative an obstruction in said cuvette.

13. A method of producing a high quality waveform representative of the amount of $CO_2$ in the expired breath of a patient which is directed to a cuvette, comprising the steps of,

generating a waveform varying with the amount of $CO_2$ in the patient's breath,

sampling the negative, or minimum concentration, peaks of said waveform continuously,

processing said waveform in conjunction with said sampling to produce an output signal representative of said breath, and

generating a signal responsive to any contamination in said cuvette to warn of said contamination to prevent false measurement of actual $CO_2$ concentration in the patient's breath.

FIG.1. WAVEFORMS, CO2 DETECTOR

FIG.3. WAVEFORMS, CO2 DETECTOR
WITH HIGH QUALITY CO2 WAVEFORN

FIG. 2